# EUROPEAN PATENT APPLICATION

(11) **EP 1 382 617 A1**
(43) Date of publication of application: **21.01.2004**
(21) Application number: 03016258.0
(22) Date of filing: 17.07.2003
(51) Int. Cl.: C07K 16/28, C12N 5/12, A61K 39/395

(54) **Monoclonal antibodies recognizing human platelet GPIIIa and GPIIb/GPIIIa and use thereof in anti-thrombotic therapy**

(30) Priority: 17.07.2002 CN 02125227
(71) Applicant: RUAN, Changgeng, Suzhou 215002 (CN); YE, George Qingwei, Mississauga, Ontario L5S 1V6 (CA)
(72) Inventor: RUAN, Changgeng, Suzhou 215002 (CN); YE, George Qingwei, Mississauga, Ontario L5S 1V6 (CA)
(74) Representative: Gervasi, Gemma, Dr.

(57) **Abstract**

The present invention relates to antibodies acting on different sites on the platelet GPIIb-IIIa complex. Said antibodies can completely block receptor function of GPIIb-IIIa complex, inhibit platelet aggregation and thereby efficiently inhibit thrombosis.

## Description

### Technical Field

The present invention relates to the field of medical immunology and more particularly to monoclonal antibodies that can recognize platelet membrane glycoproteins, hybridoma for producing the monoclonal antibodies, anti-platelet medicament containing said monoclonal antibodies and uses thereof in anti-thrombotic therapy.

### Background of The Invention

Diseases induced or complicated by thrombus are about 3 times more than oncosis. These diseases seriously threaten human health and are one of the most common reasons of human death. Accordingly, to prevent the formation of thrombus becomes highlight in the modern medical research. Platelet, as a major component of thrombus, plays a critical role in thrombosis. In normal blood circulation, platelets exist in resting status, but when the blood vessel is damaged and the subendothelial matrix such as collagen is exposed, platelets will adhere to the subendothelial matrix of the damaged vessel via direct binding membrane glycoproteins thereof to some plasma adhesive proteins such as von Willebrand factor (vWF). The platelets which adhere to matrix or are activated by some agonists produced in the process of blood coagulation and tissue injure, will change their forms, spread the pseudopodia and further release the intracellular granular contents. Concurrently, platelet membrane glycoprotein (GP) IIb-IIIa complex is activated to form the receptor of adhesive molecules, the binding of fibrinogen to which then promotes adhesion, aggregation of platelets and ultimately lead to the formation of platelet thrombus at the damaged vessel wall (Plow E.F, Ginsberg M.H. Cellular adhesion: GPIIb/IIIa as a prototypic adhesion receptor. *Progress in Haemostasis and Thrombosis*.1989; 9: 117-124). The platelet thrombosis plays a key role in pathogenesis of diseases associated with arterial thrombo-embolism, including coronary thrombosis such as acute myocardial infarction. Consequently, anti-platelet agents have become important therapy for the diseases of arterial thrombo-embolism including coronary heart disease (Becker R.C. Thrombin antagonists and antiplatelet agents. *Am J Cardiol* 1992; 69: 39E; Ruan C.G. Monoclonal antibodies and thrombotic diseases. *Science and Technology at the Frontier in China* [Ed. By Chinese Engineering Academy] 2001;Vol 4: 131-145).

Drugs currently utilized in clinics include Aspirin, Dipyridamole and Ticlopidine etc., which only function on one of the steps in the process of platelet activation and their curative effects are not as good as that of GPIIb-IIIa antagonists. GPIIb-IIIa antagonists directly inhibit the binding of fibrinogen to GPIIb-IIIa complex receptor, the final common pathway of platelet aggregation, then inhibit the formation of platelet thrombus, and accordingly have become the specific and potent anti-platelet agents and exhibited favorable prospect of application. There are three kinds of GPIIb-IIIa antagonists: monoclonal antibody such as c7E3, synthetic peptide such as Eptifibatide and non-peptide small molecule such as Tirofiban (Ruan C.G. Monoclonal antibodies and thrombotic diseases. *Science and Technology at the Frontier in China* [Ed. By Chinese Engineering Academy] 2001;Vol 4: 131-145. Verstraete M. Synthetic inhibitors of platelet GPIIb/IIIa in clinical development. *Circulation.* 2000; 101(6): 76-80).

The technique of monoclonal antibody (McAb) originated in 1975 and has brought great effects on fundamental research of biology and medical science, as well as diagnosis and therapy of diseases. However, along with the extensive application of monoclonal antibody in clinical therapy, some defects of murine monoclonal antibody agents are found that the use of murine antibody in human leads to the appearance of human anti-mouse action (HAMA); the molecule weight of antibody is so large that makes the amount of the antibody available in the target site inadequate; and low efficacy of antibody *per se,* which make curative effect unsatisfactory. To overcome these defects, humanization of the antibody is carried out, such as studies on preparation of human-mouse chimeric antibody, preparation of antibody fragment of small molecule such as single chain antibody and preparation of bifunctional antibody. 7E3, a murine monoclonal antibody against GPIIb-IIIa, was prepared by American researchers in 1983. (Coller BS, Peerschke E1, Scudder LE *et al.* A murine monoclonal antibody that completely blocks the binding of fibrinogen to platelet produces a thrombasthenic-like state in normal platelet and bind to glycoprotein IIb/IIIa. *Journal of Clinical Investigation.* 1983; 72(1): 325-328). In 1992, it was reconstructed as the Fab fragment of human-mouse chimeric antibody named c7E3. (Knight DM, Wagner C, Jordan R, *et al.* The immunogenicity of the 7E3 murine Fab monoclonal-antibody fragment variable region in dramatically reduced in humans by substitution of human for murine constant regions. *Molecular Immunology.* 1995; 32 (16): 1271-1281). Large-scale clinical investigation has manifested that c7E3 has strong ability of anti-thrombosis. The application of this Fab fragment in clinic has been approved by FDA. Nowadays it is widely used in the treatment of ischemic heart disease and has revealed the favorable future of clinical application of this kind of drugs.

However, deficiency of c7E3 consists in that it only acts on one epitope on platelet, blocks the function of receptor in a single way and thus cannot achieve complete inhibition to platelet aggregation. So it is desirable all along for a novel monoclonal antibody, which can inhibit platelet aggregation more efficiently, to be developed.

### Brief Description of the Invention

The first aspect of the invention is to provide novel monoclonal antibodies against the specific epitope of platelet membrane glycoprotein.

The second aspect of the invention is to provide novel hybridoma cells capable of producing monoclonal antibodies against the specific epitope of platelet membrane glycoprotein.

The third aspect of the invention is to provide a novel method of preparing the monoclonal antibodies against the specific epitope of platelet membrane glycoprotein.

The fourth aspect of the invention is to provide novel monoclonal antibody fragments against the specific epitope of platelet membrane glycoprotein.

The fifth aspect of the invention is to provide a novel pharmaceutical composition capable of inhibiting platelet aggregation efficiently.

### Brief Description of the Drawings

- Figure 1: shows the flow chart of preparation of hybridoma cells for excreting monoclonal antibodies R813 and Y262.
- Figure 2: shows the results of electrophoresis analysis of purified monoclonal antibodies R813 and Y262 IgG.
- Figure 3: shows the results of Western-blot analysis of monoclonal antibodies R813 and Y262.
- Figure 4: shows the results of electrophoresis analysis of the product obtained by affinity chromatography linked with R813 and Y262, wherein A represents protein marker; B represents the non-reductive product of R813; C represents protein marker; D represents the non-reductive product of Y262.
- Figure 5: shows the binding activity of monoclonal antibodies R813, Y262 to platelet, wherein 1. represents negative control; 2 represents positive control, SZ-22; 3 represents monoclonal antibody R813 and 4 represents monoclonal antibody Y262.
- Figure 6: shows the results of radioimmnoassay competitive inhibition experiment of ¹²⁵I labeled monoclonal antibodies Y262 and R813.
- Figure 7: shows the results of electrophoresis analysis of the F(ab')₂ fragments of monoclonal antibodies R813 and Y262.

### Detailed Description of the Invention

According to the first aspect of this invention, novel monoclonal antibodies against the specific epitope on the platelet membrane glycoprotein are provided.

Human platelet is adopted to immunize Balb/C mouse and a number of monoclonal antibodies (about 20) against platelet GPIIIa or GPIIb-IIIa complex are obtained. After repeated screening and testing, the monoclonal antibodies Y262, Y321, Y474 and R813 are found to be capable of inhibiting platelet aggregation efficaciously. By means of competitive radioimmunoassay of ¹²⁵I labeled monoclonal antibodies and the add-up of mean intensity of fluorescence in flowcytometry, we find that the epitopes recognized by monoclonal antibodies of the invention are different, wherein the first group of monoclonal antibodies recognizing platelet GPIIIa are selected from R813, Y88, Y128 and Y474, the second group of monoclonal antibodies recognizing platelet GPIIb-IIIa complex are selected from Y8, Y262, Y291, Y295, Y321, Y342, Y458, Y585 and Y700. The preferred monoclonal antibody of the first group is R813 and that of the second group is Y262.

According to the second aspect of this invention, novel hybridoma cell lines capable of producing monoclonal antibodies against specific epitope of platelet membrane glycoprotein are provided. Particularly the invention provides hybridoma cell lines capable of producing above monoclonal antibodies of first and second groups respectively, which are selected from hybridoma cells R813, Y88, Y128 and Y474 that produce monoclonal antibodies recognizing platelet GPIIIa and hybridoma cells Y8, Y262, Y291, Y295, Y321, Y342, Y458, Y585 and Y700 that produce monoclonal antibodies recognizing platelet GPIIb-IIIa complex, wherein the preferred hybridoma cells are R813 and Y262. The two hybridoma cells have been deposited on April 30,2002 with China Microbiological Culture Center (CGMCC), an International Deposition Authority under the Budapest treaty, having the accession numbers of CGMCC No. 0740 and CGMCC No. 0741 respectively..

According to the third aspect of this invention, a novel method for preparing the monoclonal antibodies against specific epitopes on platelet membrane glycoprotein is provided. Said method includes using humans platelet as immunogen to immunize Balb/C mouse, fusing the spleen cells of immunized mouse with myeloma cells-SP2/0-Ag14 to prepare hybridoma cells capable of producing monoclonal antibodies recognizing platelet GPIIIa or GPIIb-IIIa complex, identifying the epitopes of the monoclonal antibodies by means of ELISA, FCM, Western-blot and affinity chromatography, investigating the binding activity of monoclonal antibodies to platelet and preparing ascites and purifying monoclonal antibodies.

According to the fourth aspect of this invention, novel F(ab')₂ fragments of monoclonal antibodies against specific epitope on platelet membrane glycoprotein, are provided. Papain is used to digest monoclonal antibodies R813 and Y262 to prepare the F(ab')₂ fragments. After identifying the fragments with SDS-PAGE, the inhibition activities of the fragments and their "cocktail" are investigated.

According to the fifth aspect of this invention, a novel pharmaceutical composition that efficiently inhibits platelet aggregation is provided, comprising one or more monoclonal antibodies of this invention and if required, pharmaceutically acceptable carriers or adjuvant.

In a preferred embodiment of this invention, monoclonal antibodies R813 and Y262 recognizing different epitopes are screened out by means of competitive radioimmunoassay of ¹²⁵I labeled monoclonal antibody and the add-up of mean intensity of fluorescence in flowcytometery. Combined use of R813 and Y262 is found to completely inhibit platelet aggregation induced by ADP and allow low dosage of antibody to be used (the gross concentration is about 6~7ug/ml). The experiment results indicates that R813 and Y262 act on different sites of platelet GPIIb-IIIa complex and the "cocktail" of these two antibodies completely blocks the binding of fibrinogen to platelet GPIIb-IIIa receptor and thus inhibits platelet aggregation. Additionally, the F(ab')₂ fragments of these two monoclonal antibodies still inhibit platelet aggregation completely, biological activities of which exceed that of c7E3 (tradename is Reopro). It is originative in the world using the "cocktail" of two monoclonal antibodies to completely block the receptor function of platelet membrane GPIIb-IIIa. Reopro acts on only one epitope of platelet GPIIb-IIIa complex, blocks the receptor in a single way and the dosage needed to achieve 50% inhibition of platelet aggregation is 5.5ug/ml. While, the "cocktail" of the invention acts on two different epitopes of platelet GPIIb-IIIa complex and completely inhibits fibrinogen to bind with receptor sterically, the dosage for 50% inhibition is only 3.3ug/ml. The "cocktail" not only decreases the dosage of monoclonal antibodies substantially but also greatly increases the inhibition to platelet aggregation, the bioactivity exceeds that of Reorpo (c7E3). To alleviate HAMA reaction induced by monoclonal antibodies, and to reduce molecular weight and increase the antibody concentration at target site, monoclonal antibodies R813 and Y262 are reconstructed by enzymolysis to prepare F(ab')₂ fragments (There are studies showing that the HAMA reaction from F(ab')₂ fragment of murine monoclonal antibody is no more severe than that from chimeric antibody and it has excellent affinity thus can be used in clinic directly.). In vitro studies showed that combined use of the F(ab')₂ fragments of these two antibodies is still capable of completely blocking the function of platelet GPIIb-IIIa receptor and inhibiting platelet aggregation.

The advantages of the invention consist in that monoclonal antibodies R813 and Y262 that act on different epitopes on platelet GPIIb-IIIa complex and inhibit platelet aggregation efficiently, are developed; the "cocktail" combining said two antibodies is capable of blocking platelet GPIIb-IIIa receptor and inhibiting platelet aggregation completely, the biological activity of which exceeds that of Reopro, a well-known anti-platelet agent, and allows low dosage to be used; the F(ab')₂ fragments prepared by enzymolysis of antibodies, remain the bioactivity, meanwhile bring down the molecular weight of antibodies and lead to more antibodies available at target site; removal of Fc decreases HAMA reaction and reduces damage of platelet induced by binding with reticuloendothelial system. In addition enzymolysis is simple to operation and low-cost. In view of the above-mentioned potent and efficient bioactivity, low cost and simple operation, the invention thus exhibits tremendous anti-thrombotic potentiality and market prospect.

The invention will be illustrated hereinafter in more detail on the basis of following unlimited examples.

### Example 1. Preparation of hybridoma cells that produce monoclonal antibodies R813 and Y262

### 1.1 Material:

Balb/C mice were purchased from Charles River Laboratories Inc. Canada, SP2/0-Ag14 mouse myeloma cell, myelocytoma cell (K562), acute lymphosarcoleukemia cell (MOLF-4) and acute T-cell leukemia cell of human (Jurkat E-6) were purchased from ATCC lnc. USA, D-MEM/F₁₂, RMP11640, L-Glutamine, MEM non-essential, HT Supplement, HAT Supplement, Antibiotic-Antimycotic were all purchased from GIBCO Inc., 8-Azaguanie was purchased from Sigma, 50% PEG 1500 Solution was purchased from ROCH Inc., Subclass Identification of Mouse IgG Kit was purchased from Biorad Inc., other reagents were all analytical reagents purchased from domestic companies.

### 1.2 Methods:

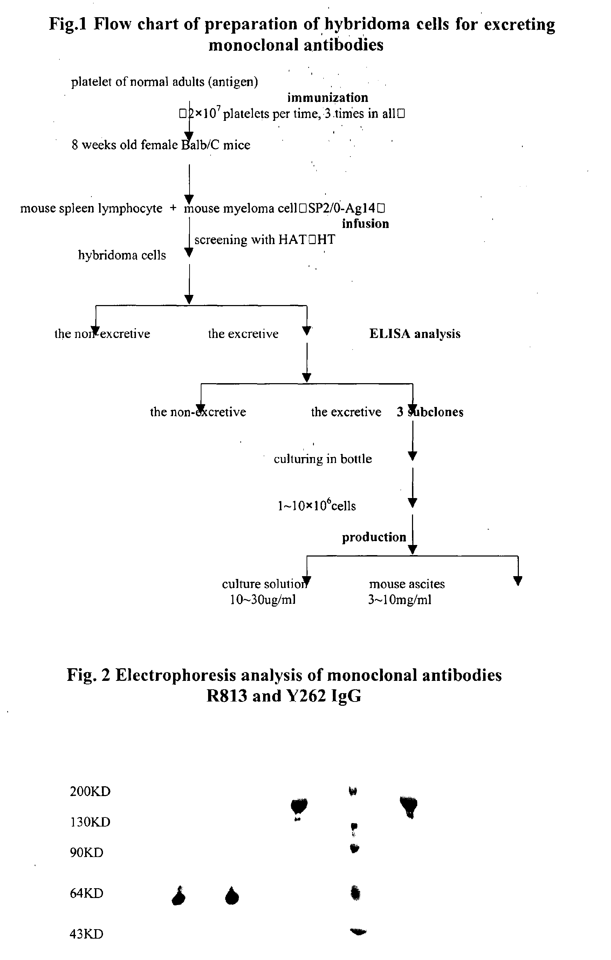

Platelet was extracted from anticoagulated normal human whole blood, washed with TEN for 3 times, suspended with PBS and adjusted the platelet concentration to 1x10⁸ cell/ml, then 0.2ml platelet was injected into the abdominal cavity of 8 weeks old female Balb/C mice. After 4 weeks this operation is repeated, another 4 weeks later 0.2ml platelet suspension was injected into tail veins, and 3 days later the mice were sacrificed. The spleen was taken out to prepare spleen cell suspension. 10⁸ spleen cells were fused with 10⁷ SP2/0-Ag14 cells in their logarithmic growth phase in accordance with conventional methods. The cells were cultivated with HAT supplement and HT supplement in turn each for 2 weeks then changed to common complete media. During this period the cell clones were detected with ELISA using platelet coated immunoassay plate and more than 20 hybridoma cell clones were found to have remarkable binding reactivity with platelet. Subcloned these positive and selected hybridoma cell clones according to limiting dilution method for 3 times and transferred the clones to 24-well cultivating plate and bottle, then ascites was prepared (Fig. 1). 12 cell strains obtained as above, which secrete anti-platelet monoclonal antibodies steadily, are R813, Y8, Y88, Y128, Y262, Y295, Y291, Y321, Y342, Y 458, Y474, Y585 and Y700. Investigation of reactions between these antibodies and human red blood cell, white blood cell, K562, MOLF-4 and Jurkat E6-1cell by flow cytometry showed that all antibodies had no cross-linked reactivity with these cells. Besides, karyotype analysis of cells producing these antibodies indicated that they are all hybridoma cells. With the subclass identification kit (Biorad) we found that monoclonal antibodies R813, Y128, Y321, Y458, Y474, Y585 and Y700 belong to IgG₁, Kappa subclass, Y8 and Y295 belong to IgG₂ₐ, Kappa subclass, Y88, Y262 and Y291 belong to IgG_{2b}, Kappa subclass. Observation of the preferred hybridoma cells R813 and Y262 for several years demonstrated capability of these two cell strains to secrete monoclonal antibody of high valence steadily. Up to now, productive cell library of these two cell strains has been constructed.

### Example 2. Preparation and purification of monoclonal antibodies R813 and Y262 2.1 Material:

Pristane was purchased from Sigma Inc., Balb/C mice were purchased from Shanghai Experimental Animal Research Center, Protein G affinity chromatography column (1.0 ml, ready for use) was purchased from Pharmacia Inc., other reagents were all analytical reagents purchased from domestic company.

### 2.2 Methods:

1ml pristane was injected into the abdominal cavity of 10 weeks old male Balb/C mice to make them allergic, 1~2 weeks later, 1~10x10⁶ hybridoma cells of R813 and Y262 were respectively injected into the abdominal cavity of the allergen-challenged mice, around a week later the ascites was collected, which was then dialyzed against 0.02 M PB solution (PH7.0) at 4□ over night, after centrifugation at 10000rpm for 15 minutes at 4□ to remove precipitates and the subsequent filtration with a filter of 0.2um, the filtrate was applied to protein G column in an amount of 1ml at a time, 0.02 M PB solution (pH7.0) was used to equilibrate the column and wash away the needless protein, followed by eluting the IgG with 0.1M Gly-HCl solution pH2.7. The eluate was collected and dialyzed against PBS, then after condensation and being quantified, the eluate was identified with 10% SDS-PAGE. The results showed the concentration of antibody in ascites was 8mg/ml and the purity of antibodies was above 90% after purification. (Fig 2)

### Example 3. The study on the binding activity of monoclonal antibodies R813 and Y262 with platelet

### 3.1 Material:

Platelet was extracted from whole blood of normal adult donors, GAM-FITC was purchased from Huamei Inc. in Shanghai, GAM-HRP was purchased from Sigma Inc., CNBr-Sepharose 4B was purchased from Pharmacia Inc., standard protein molecule weight marker was purchased from Sibasi Inc. in Shanghai, it consisted of the protein with molecule weight 43KD, 66KD, 97KD, 130 KD and 200KD, other reagents were all analytical reagents purchased from domestic companies.

### 3.2 Methods:

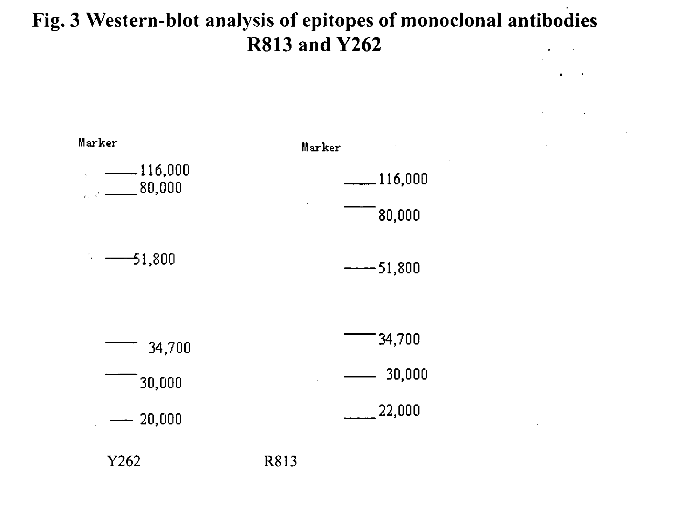

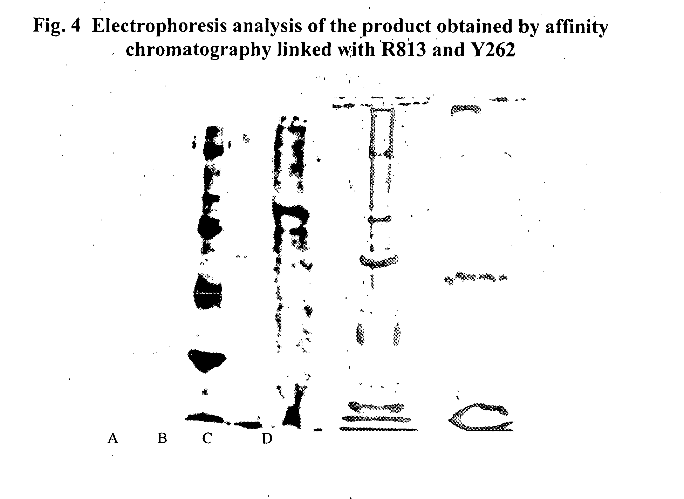

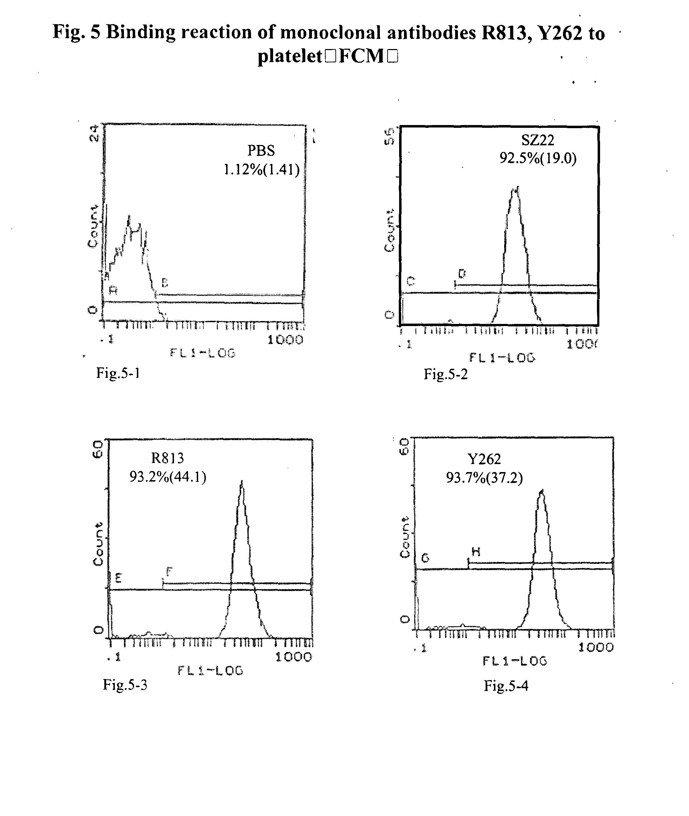

(1) Study on the binding activity of monoclonal antibodies R813 and Y262 with platelet by ELISA: platelet rich plasma (PRP) was extracted from anticoagulated blood of healthy subjects, washed with TEN solution for 3 times, then concentration was adjusted to 5x10⁵/ml. 0.1 ml platelet was dispensed in each well of ELISA plate and the plate was placed at 4□ over night, followed by 2 hours of blocking at 37□ with 2%BSA-PBS, immobilized with 0.5% glutaraldehyde, washed with 0.01%Tween 20-PBS, GAM-HRP acting as secondary antibody and color was developed with OPD, then the absorbance at 490nm was tested by conventional ELISA. Mouse-IgG was used as negative control and antibody SZ-22 (against GPIIb) as positive control. The results showed the excellent binding activity of R813 and Y262 with platelet.
(2) Study on the binding activity of monoclonal antibodies R813 and Y262 with platelet by flowcytometry: 5ul PRP (the platelet concentration is 5x10⁸/ml) was mixed with 50ul ascities of antibodies R813 and Y262 (1:100 diluted with PBS) respectively, the mixture was placed at room temperature for 30 minutes, after washing, the GAM-FITC was added in then the specimen were tested with flowcytometer (Coulter EPICS^{R}, XL). Mouse-IgG was used as negative control and equally diluted antibody SZ-22 as positive control. The results showed that antibodies R813 and Y262 were capable of binding to platelet. (Fig 5.)
(3) Determination of binding sites of antibodies R813 and Y262 with platelet membrane glycoprotein by Western-blot assay: PRP was extracted from EDTA-anticoagulated whole blood, washed with TEN solution for three times and platelet concentration was adjusted to 2x10⁸/ml. Platelet was lysed when 1mM PMSF and 1% TritonX-100 were added in. After centrifugation, the supernatant was dispensed in plate in an amount of 60ul/well, and non-reductive SDS-PAGE was carried out under 5%-15% logarithmic gradient, then the protein was transferred into nitrocellulose membrane. After blocking with 2%BSA-PBS, the membrane was cut into stripes and was incubated respectively with antibodies R813 and Y262 at 37□ for 2 hours with GAM-HRP as the secondary antibody. Finally chloronaphthalenephenol was added to develop color. Mouse-IgG was used as negative control and antibody SZ-22 as positive control. The results showed that R813 acted on platelet GPIIIa, while no reaction took place with Y262. (Fig 3.)
(4) Determination of binding sites of antibodies R813 and Y262 with platelet membrane glycoprotein by affinity chromatography: CNBr-Sepharose 4B gel was swollen with 1mM HC1 for 4 hours then washed with coupling buffer for 3 times. Purified antibodies R813 and Y262 were sufficiently dialyzed and then applied to the prepared gel respectively. The mixture was vibrated for 16 hours at 4□, after that it was washed with coupling buffer in tundish, saturated with 1mM ethanolamine for 2 times, each for one hour, then washed with coupling buffer, 25mM Tris-HCl and TBS in turn, at last it was loaded into the column. The platelet lysate was prepared according to method (3). The lysate of 2×10⁹ platelet was applied to the column repeatedly and eluted with 0.05M diethyl amine-5mM EDTA buffer. The eluate was dialyzed against PBS and after concentration it was identified with 10% SDS-PAGE. The results showed that R813 recognized platelet GPIIIa, while Y262 recognized platelet GPIIb-IIIa complex (Fig 4.)

### Example 4. Different epitopes of platelet GPIIb-IIIa receptor that the antibodies R813 and Y262 respectively recognize

### 4.1 Material:

¹²⁵I was purchased from China Nucleus Power Company, Sephadex G-25 gel was purchased from Pharmacia Inc., other reagents were all analytical reagents purchased from domestic companies.

### 4.2 Methods:

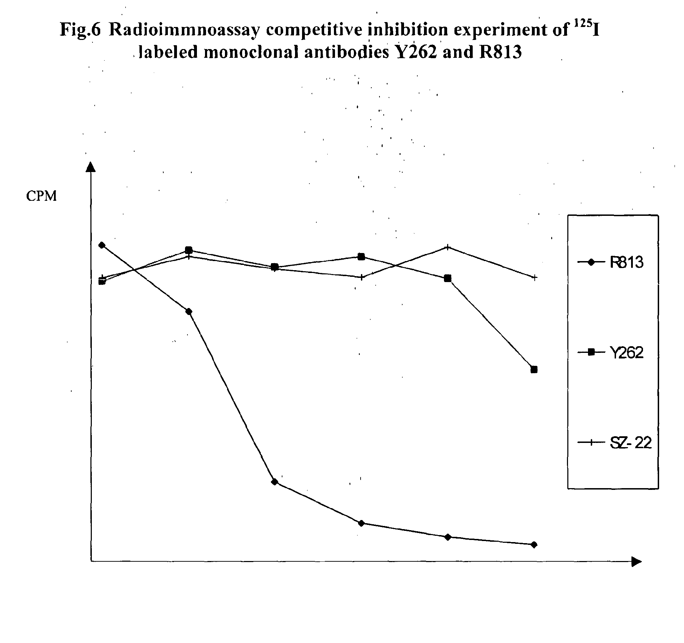

Competitive radioimmunoassay of ¹²⁵I labeled monoclonal antibody R813: 30ug of R813 (1mg/ml) was mixed with 10ul 0.1M PB pH 7.5, 1mGi ¹²⁵I and 10ul ChT (5mg/ml), the mixture was placed at room temperature for 1 minute, then 20ul Na₂S₂O₈ (20mg/ml) was added and placed for another minute, at last the reaction was ended with the addition of 100ul 0.5% BSA-PBS. The reaction mixture was applied to Sephadex G-25 gel column pre-equilibrated with 0.5% BSA-PBS, and eluted with the same solution. The eluate was collected in the unit of 10 drips per vial. Then the radioactivity of each vial was tested withycounter and the first radioactivity peak was collected. Platelet was coated on ELISA plate according to previous method. Fixed amount of 1²⁵I-R813 was mixed with different concentrations of murine IgG, R813 and Y262 respectively, after that the mixtures were dispensed into the ELISA plate, followed by 2 hours of incubation at 37□ and 3 washes, finally the radioactivity was tested with Y counter. Negative control was mouse IgG. The results showed that the radioactivity dropped with the increase of concentration of R813, while it remained unchanged as concentrations of Y262 and murine IgG varied, which indicated that R813 and Y262 recognized different epitopes (Fig 6.)

### Example 5. The synergetic action of antibodies R813 and Y262 in blocking platelet GPIIb-IIIa complex and in inhibiting platelet aggregation

### 5.1 Material:

ADP was purchased from Lizhudongfeng Biological Product Inc. in Shanghai, GAM-FITC was purchased in Huamei Inc. in Shanghai, other reagents were all analytical reagents purchased from domestic companies.

### 5.2 Methods:

(1) The add-up of mean intensity of fluorescence by flow cytometry: 5ul PRP was mixed with R813, Y262 and mixture of both respectively and placed at room temperature for 30 minutes. GAM-FITC was added after wash, then the mixture was detected with flowcytometer. Mouse IgG was used as negative control. The results showed that the mean intensity of fluorescence of combination of R813 and Y262 corresponded to the sum of that of two individual antibodies, and which indicated that the two antibodies can simultaneously act on their respective epitopes, thus synergetically blocked the platelet GPIIb-IIIa complex. (Table 1.)

**Table 1.**

| The add-up of mean fluorescent intensity of R813 and Y262 by flow cytometry | | |
|---|---|---|
| Antibody | Percentage of positive cell | (%) Mean fluorescent intensity |
| PBS | 3.22 | 2.61 |
| M-IgG | 6.48 | 3.26 |
| SZ-22 | 90.2 | 83.1 |
| R813 | 87.7 | 185.7 |
| Y262 | 73.6 | 130.9 |
| R813+M-IgG | 86.4 | 174.8 |
| R813+SZ-22 | 92.3 | 255.4 |
| R813+R813 | 87.6 | 180.1 |
| R813+Y262 | 88.1 | 338.5 |

(2) The synergetic action of antibodies R813 and Y262 in inhibiting platelet aggregation induced by ADP: PRP was prepared from citrate-anticoagulated whole blood of healthy adults, then 200ul PRP was mixed with R813, Y262 and the mixture of both respectively, after incubated at 37□ for 5 minutes, ADP was added (final concentration was 5uM) and the aggregation rate induced be tested. Mouse IgG was used as negative control and Reopro as positive control. The results showed that R813 and Y262 inhibited platelet aggregation efficiently, and combined use of both under the same concentrations exhibited enhanced inhibition, the platelet aggregation thus can be completely blocked, its anti-thrombotic activity was superior to that of Reopro.

Besides, the dosage for 50% inhibition was found to be less than that of Reopro. (Table2. 3. 4.)

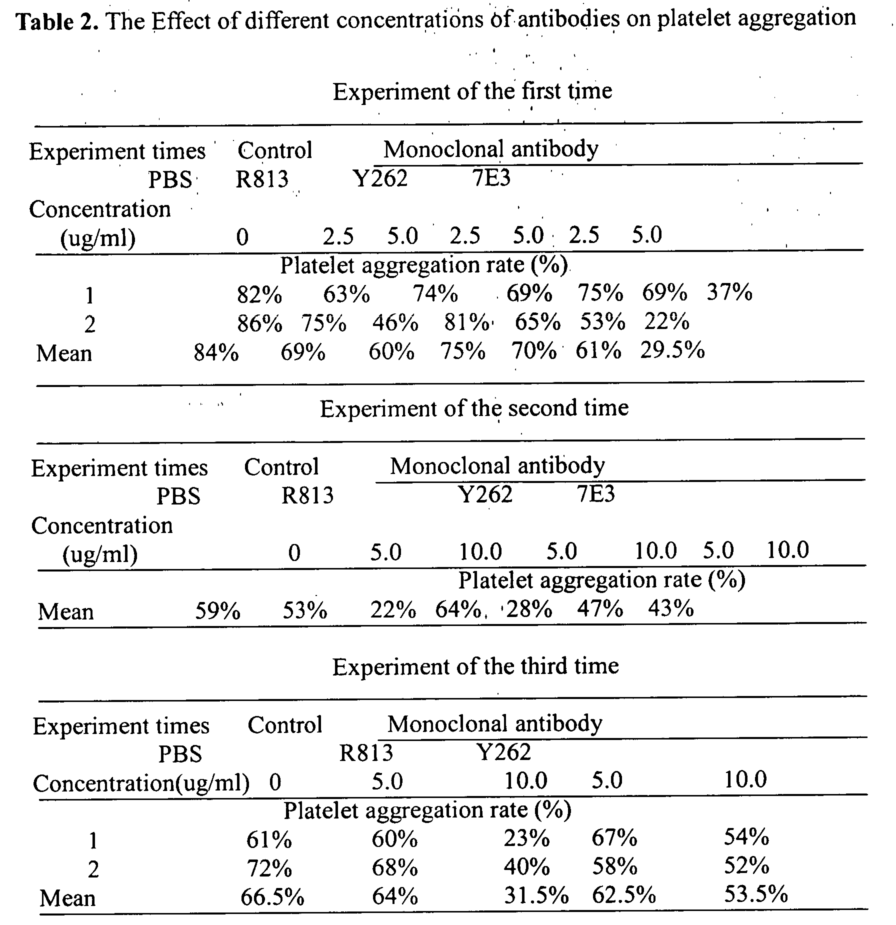

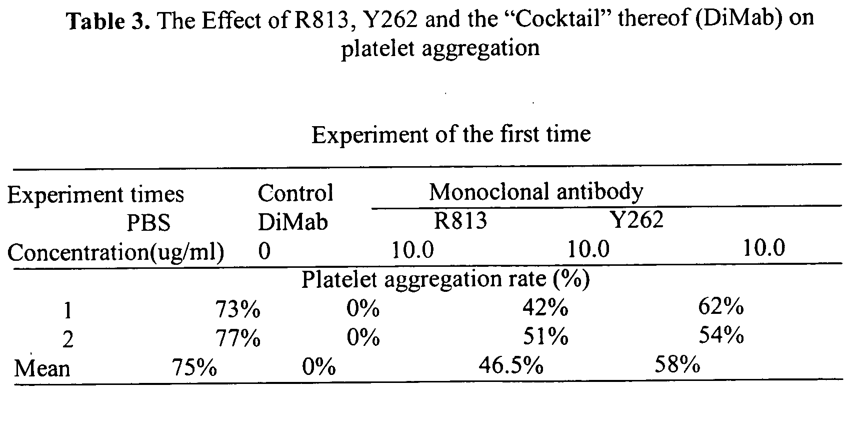

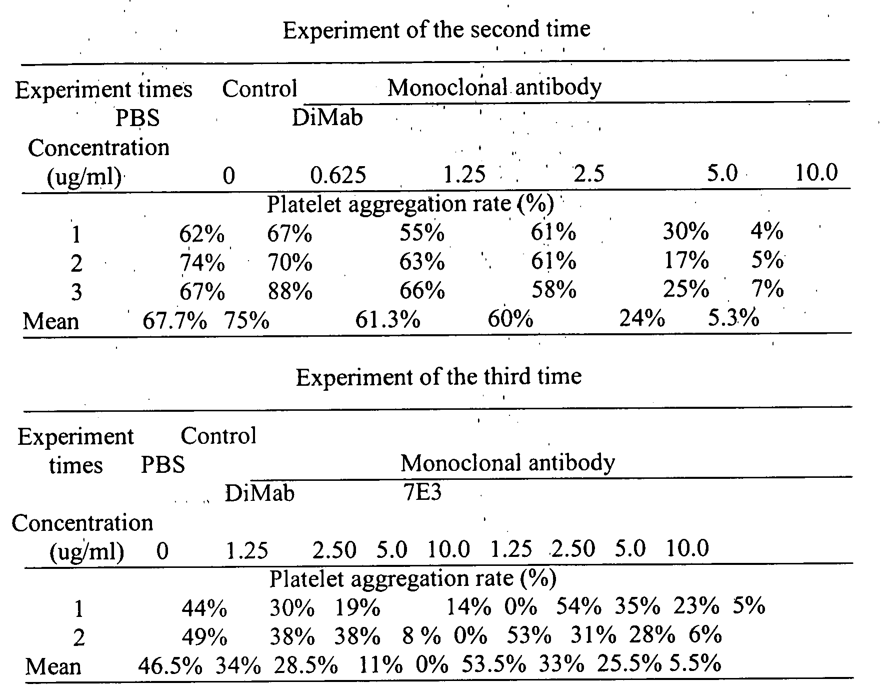

**Table 4.**

| 50% effective dosage of "cocktail" (DiMab) and 7E3 for platelet aggregation | | |
|---|---|---|
| | DiMab | 7E3 |
| ug/ml | 3.13 | 5.26 |

### Example 6. The preparation and identification of F(ab')₂ fragments of antibodies R813 and Y262

### 6.1 Material:

Papain was purchased from Lizhudongfeng Biological Product Inc. in Shanghai, DEAE-52 gel was purchased from Sibasi Inc. Biochemistry Research Institute, in Shanghai, DTT was the product of Fluka Inc. other reagents were all analytical reagents purchased from domestic companies.

### 6.2 Methods:

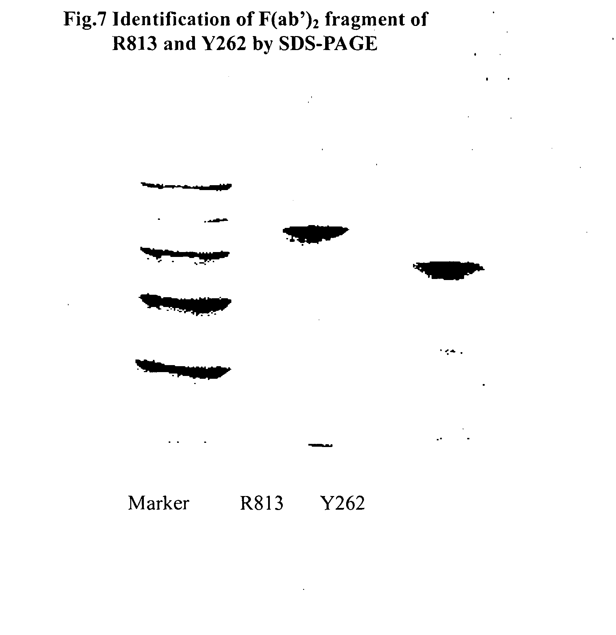

(1) Preparation and identification of F(ab')₂ fragments of R816 and Y262 : R813 and papain were mixed uniformly in a ratio of 5:1, and reacted at 4□ for 24 hours, then the reaction was ended by addition of iodoacetamide (final concentration was 80mM). The digested product was dialyzed against 0.01 M Tris-HCl PH8.0 and then was applied to DEAE-52 gel column. 0~0.3 M NaCl and 0.01M Tris-HCl PH8.0 was used as eluent and the first eluted peak was collected. Y262 and papain were mixed uniformly in a ratio of 80:1, and reacted at 4□ for 35 minutes. Then papain and Y262 in a ratio of 1:160 were further added, and reacted at 4□ for another 35 minutes. Subsequent process was the same as that of R813 but the second eluted peak instead of the first one was collected. The collected products were treated by reduced and non-reduced SDS-PAGE under 10% gradient degree. The results showed that the digestive products were F(ab')₂ fragments of antibodies (Fig 7.)
(2) Inhibition of platelet aggregation induced by ADP, by F(ab')₂ fragments of R813 and Y262: The method was the same as above and the results showed that the F(ab')₂ fragments of R813 and Y262 still had synergetic action of completely inhibiting platelet aggregation. (Table 5.)

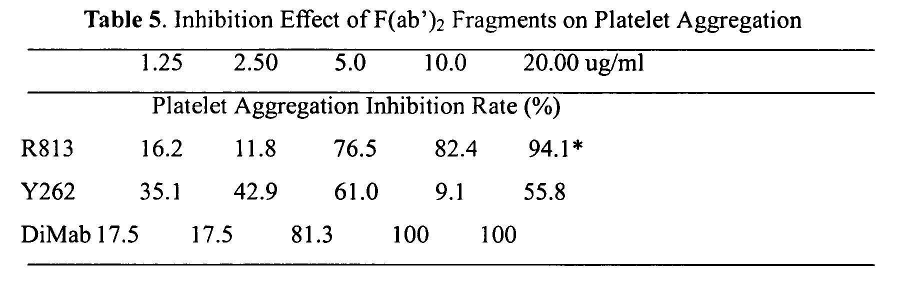

The invention has been described in great detail in foregoing specification and Examples, and it is believed that various alternations and modifications of the invention will become apparent, without deviating the principles and spirits of the invention, to these skilled in the art, in light of disclosure of the present specification and claims. Therefore, it is intended that all such alternations and modifications are included in the scope of protection in appended claims.

## Claims

1. A monoclonal antibody against platelet GPIIIa, which is produced by hybridoma cell lines selected from R813, Y88, Y128 and Y474.

2. A monoclonal antibody against platelet GPIIb-IIIa complex, which is produced by hybridoma cell lines selected from Y8, Y262, Y291, Y295, Y321, Y458, Y585 and Y700.

3. Monoclonal antibody according to claim 1 or 2., which comprises IgG Fab, F(ab')₂ fragments, single chain antibody, chimeric antibody, humanized, antibody and gene engineering antibody of said monoclonal antibody.

4. A number of hybridoma cell lines in claim 1 and 2 that produces monoclonal antibody specifically blocks the receptor function of GPIIb-IIIa complex, which are selected from Y262, Y321, Y474 and R813.

5. Hybridoma cell line described in claim 4, which is R813, CGMCC No. 0740.

6. Hybridoma cell line described in claim 4, which is Y262, CGMCC No. 0741.

7. A pharmaceutical composition, which inhibits platelet aggregation efficiently, **characterized in that** it contains one or more monoclonal antibodies selected from the following two groups:
(1) monoclonal antibodies against platelet GPIIIa, and/or
(2) monoclonal antibodies against platelet GPIIb-IIIa complex, and pharmaceutically acceptable carriers or excipients.

8. Pharmaceutical composition as described in claim 7, wherein monoclonal antibody against platelet GPIIIa is selected from R813, Y88, Y128 and Y474.

9. Pharmaceutical composition as described in claim 7, wherein monoclonal antibody against platelet GPIIb-IIIa complex is selected from Y8, Y262, Y291, Y295, Y321, Y458, Y585 and Y700.

10. Pharmaceutical composition as described in claim 7, **characterized in that** it contains monoclonal antibodies R813 and Y262.

11. Pharmaceutical composition as described in claim 10, wherein the ratio of monoclonal antibody R813 to Y262 is 1:1.

12. Process for preparing monoclonal antibody as described in any one of claims 1 to 3, comprising following steps:
(1) immunizing Balb/c mouse using human platelet to prepare hybridoma cell line that produces anti-platelet antibody;
(2) cultivating the hybridoma cell line to produce monoclonal antibody;
(3) purifying obtained monoclonal antibody and identifying its epitopes; and optionally
(4) using papain to digest, and to purify the digestive products to acquire fragment of said antibody.
